# EUROPEAN PATENT APPLICATION

(11) **EP 1 524 319 A1**
(43) Date of publication of application: **20.04.2005**
(21) Application number: 03078246.0
(22) Date of filing: 13.10.2003
(51) Int. Cl.: C12Q 1/18, G01N 33/50

(54) **Method of classifying chemical agents and identifying cellular targets thereof**

(71) Applicant: Nederlandse Organisatie voor Toegepast-Natuurwetenschappelijk Onderzoek TNO, 2628 VK Delft (NL)
(72) Inventor: Montijn, Roy Christiaan, 1024 NL Amsterdam (NL); Schuren, Frank Henri Johan, 3906 ZK Veenendaal (NL)
(74) Representative: Prins, Adrianus Willem, Mr. Ir.

(57) **Abstract**

The present invention is in the field of characterization of antibiotic compounds, e.g. such as compounds useful for treating pathogenic infections in mammals and identification of cellular targets thereof. The invention further relates to a method of classifying antibiotic compounds, to methods for determining their modes of action and for identifying new antibiotic targets based on classification schemes comprising clustering according to similarity the biochemical response profiles (e.g. gene expression pattern) of an organism exposed to said compounds.

## Description

### FIELD OF THE INVENTION

The present invention is in the field of characterization of antibiotic compounds, e.g. such as compounds useful for treating pathogenic infections in mammals and identification of cellular targets thereof. The invention further relates to a method of classifying antibiotic compounds and to methods for determining their modes of action.

### BACKGROUND OF THE INVENTION

Outbreaks of foodborne and waterborne infectious disease are on the increase around the world and are a serious public health threat. To make matters worse, the percentage of micro-organisms that are resistant to antibiotics is also increasing rapidly, and researchers are becoming increasingly concerned about crossover resistance associated with the food we eat and the water we drink. Of specific concern is resistance to methicillin in strains of *Staphylococcus aureus* (MRSA) and resistance to the antibiotic of "last-resort" vancomycin in strains of Enterococcus bacteria (VRE) and the spread thereof to other organisms.

In the past, the solution to bacterial resistance has been primarily dependent on the development of clinically viable anti-microbial agents. Apart from the discovery of natural antibacterial peptides from plants and animals, there have been few new antibiotics developed in recent years. As a result, little is presently expected of the traditional antibiotic screening methods, based on direct measurements in living cells of the inhibitory capacities of particular compounds.

In light of the foregoing it would be a significant advancement in the art to provide a method of identifying the molecular target of a lead compound, i.e. an antibiotic compound that is non-toxic to humans. It would be a further advancement if the method was capable of identifying new molecular targets for antibiotic compounds.

### SUMMARY OF THE INVENTION

The present invention is based on the insight that intrinsic changes in the biochemical composition of a micro-organism which result from external stimuli received by that micro-organism from its surrounding environment are characteristic for the nature of the stimulus. Therefore, by measuring these intrinsic changes, the micro-organism may be used as a sensor to determine the condition of its environment and, more specifically, the nature of the stimulus. An example of such a stimulus is a chemical substance that is capable of inhibiting the growth or even of killing a micro-organism, such as an antibiotic compound. It has now surprisingly been found that the reaction of a micro-organism to substance exposure and the specific way in which that substance affects the growth of the micro-organism can be used to classify those substances, e.g. either by mode of action of the substance, by molecular target of the substance and/or by metabolic route affected by the substance.

The present invention now utilizes this finding to provide, in a first aspect, a method of producing a classification scheme for chemical compounds comprising clustering according to similarity the biochemical response profiles of an organism as determined by exposing said organism to a multitude of individual chemical compounds. In a preferred embodiment, said clustering according to similarity comprises principal component analysis. In another preferred embodiment said chemical compounds are antibiotic compounds. In yet another preferred embodiment said multitude of individual chemical compounds comprises reference compounds with known mode of action and wherein said profiles are reference response profiles clustered according to similarity in mode of action.

In another aspect, the present invention provides a classification scheme for chemical compounds obtainable by a method of the invention.

In a further aspect the present invention provides a method of classifying a chemical compound, comprising the steps of providing a classification scheme for chemical compounds by performing a method of the invention of producing a classification scheme for chemical compounds as described above, exposing an organism to said chemical compound, determining the biochemical response profile of said organism, and determining in said scheme the clustered position of said biochemical response profile.

In yet a further aspect the present invention provides a method of classifying an antibiotic compound according to mode of action comprising the steps of providing a classification scheme for chemical compounds by performing a method of producing a classification scheme for chemical compounds as described above, wherein the chemical compound is an antibiotic compound, wherein the multitude of individual antibiotic compounds comprises reference compounds with known mode of action and wherein the profiles are reference response profiles clustered according similarity in mode of action, said method comprising the further steps of exposing an organism to said antibiotic compound, determining the biochemical response profile of said organism, determining in said scheme the clustered position of said biochemical response profile, and assigning to said antibiotic compound the mode of action of the corresponding cluster of reference response profiles or assigning to said antibiotic compound a new mode of action in case said position is outside any known cluster of reference response profiles.

In yet a further aspect, the present invention provides a method of analysing a mode of action of an antibiotic compound comprising the steps of performing a method of classifying an antibiotic compound according to the invention, wherein the biochemical response profile is a transcription response profile, thereby determining for said antibiotic compound the clustered position of the transcription response profile, identifying from said transcription response profile the genes of said organism of which the expression is substantially affected by exposure to said antibiotic compound and which characterize said clustered position, and determining from the identity of said genes the cellular process affected by said antibiotic compound.

In yet a further aspect the present invention provides a method of identifying a new mode of action of an antibiotic compound comprising the steps of performing a method of classifying an antibiotic compound according to the invention, wherein the biochemical response profile is a transcription response profile, selecting an antibiotic compound of which the corresponding transcription response profile has a clustered position outside any known cluster of reference response profiles, identifying from the transcription response profile of said selected compound the genes of said organism of which the expression is substantially affected by exposure to said selected antibiotic compound and which characterize said clustered position, and determining from the identity of said genes the cellular process affected by said selected antibiotic compound thereby identifying the new mode of action.

In yet a further aspect the present invention provides a method of identifying the molecular target of an antibiotic compound comprising the steps of performing a method of analysing a mode of action of an antibiotic compound as described above or performing a method of identifying a new mode of action of an antibiotic compound as described above, selecting a group of potential molecular targets characterizing the cellular process affected by the antibiotic compound, and identifying the molecular target of the antibiotic compound in the group of potential molecular targets.

In yet a further aspect the present invention provides a method of identifying a new metabolic route comprising the steps of performing the method of classifying a chemical compound as described above, wherein the biochemical response profile is a transcription response profile, wherein said multitude of individual chemical compounds comprises reference compounds with known mode of action and wherein said profiles are reference response profiles clustered according similarity in mode of action, selecting a chemical compound of which the corresponding transcription response profile has a clustered position outside any known cluster of reference response profiles, identifying from said transcription response profile the genes of said organism of which the expression is substantially affected by exposure to said chemical compound and which characterize said clustered position, and determining from the identity of said genes the cellular process affected by said chemical compound thereby identifying a new metabolic route.

### DESCRIPTION OF THE FIGURES

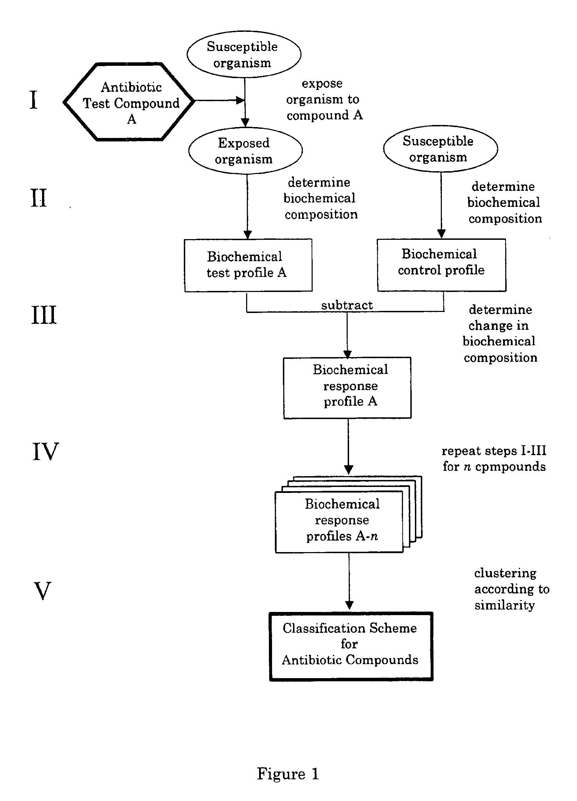

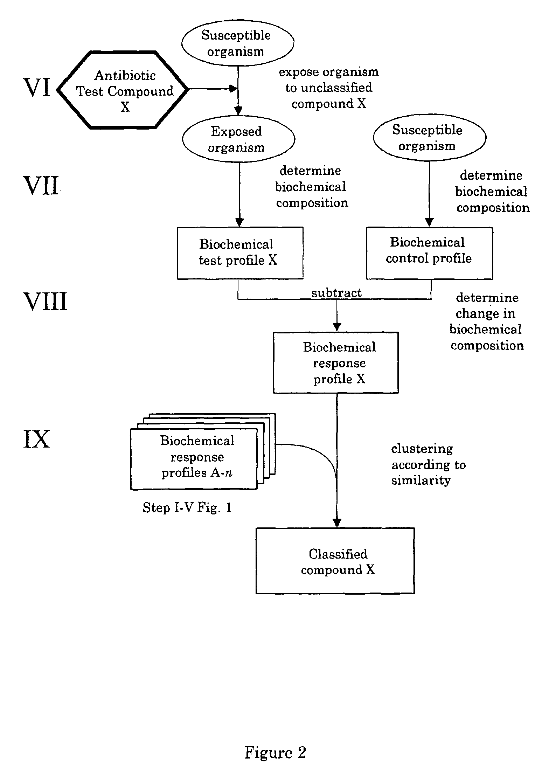

Figure 1 shows a flow chart of the method of producing a classification scheme for chemical compounds essentially as claimed in claim 1.
Figure 2 shows a flow chart of the method of classifying a chemical compound essentially as claimed in claim 8.

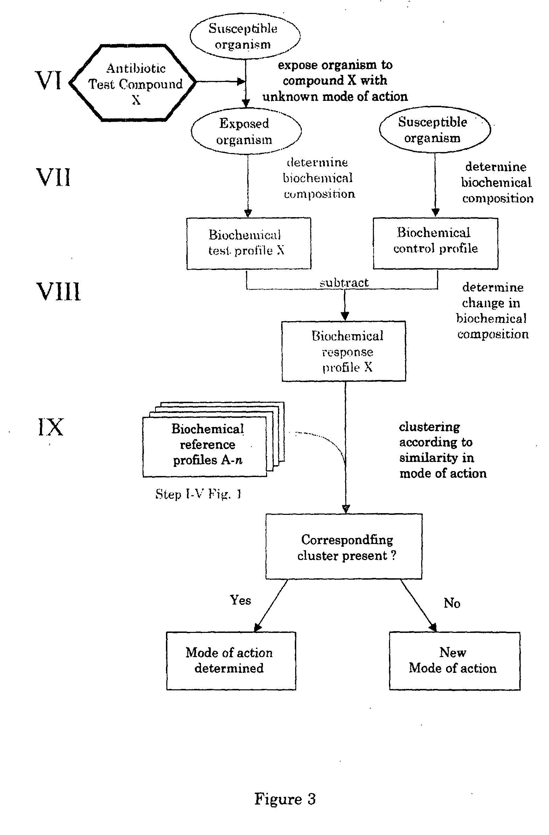

Figure 3 shows a flow chart of the method of classifying an antibiotic compound according to mode of action essentially as claimed in claim 9

### DETAILED DESCRIPTION OF THE INVENTION

The term "item" as used herein refers to anything classifiable by a method of the present invention and includes both a chemical compound, e.g. an antibiotic compound, and a biochemical response profile, e.g. a transcription response profile or a reference profile.

The term "classifying" is used in its art-recognized meaning and thus refers to arranging or ordering items, *i.e.* chemical compounds or response profiles, by classes or categories or dividing them into logically hierarchical classes, subclasses, and sub-subclasses based on the characteristics they have in common and/or that distinguish them. In particular "classifying" refers to assigning, to a class or kind, an unclassified chemical compound or response profile. A "class" then being a grouping of items, based on one or more characteristics, attributes, properties, qualities, effects, parameters, etc., which they have in common, for the purpose of classifying them according to an established system or scheme.

The term "classification scheme" is used in its art-recognized meaning and thus refers to a list of classes arranged according to a set of preestablished principles, for the purpose of organizing items in a collection or into groups based on their similarities and differences.

The term "clustering" refers to the activity of collecting, assembling or uniting into a cluster or clusters items with the same or similar elements, a "cluster" referring to a group or number of the same or similar items gathered or occurring closely together. "Clustered" indicates that an item has been subjected to clustering.

The term "clustered position" refers to the location of an individual item in amongst a number of clusters, said location being determined by clustering said item.

The process of clustering used in a method of to the present invention may be any mathematical process known to compare items for similarity in characteristics, attributes, properties, qualities, effects, , etc., through data from measurable parameters. Statistical analysis, such as for instance multivariance analysis, or other methods of analysis may be used. Preferably methods of analysis such as self-organising maps, hierarchical clustering, multidimensional scaling, principle component analysis, supervised learning, k-nearest neighbours, support vector machines, discriminant analyse and/or partial least square methods are used. In a most preferred embodiment of a method of the present invention principal component analysis is used to cluster items according to similarity.

A "biochemical response profile" as used herein refers to a set of data, usually in graph or table form, that represents the qualitative or quantitative change in the biochemical composition of an organism resulting from a stimulus, i.e. an exposure of that organism to a chemical compound. Throughout this description with said biochemical composition is meant the composition of the various (bio)chemical compounds that together form the cell and with said change in the biochemical composition is meant the composition before the stimulus minus the composition after the stimulus, corrected for general test influences.

A "transcription response profile" as used herein refers to a set of data, usually in graph or table form, that represents the qualitative or quantitative change in the mRNA pool of an organism resulting from a stimulus, *i.e.* an exposure of that organism to a chemical compound. A "transcription response profile" may suitably be obtained by transcription profiling, the method that uses 10 to thousands of different DNA probes arranged in microarrays, with each DNA representing a different gene or part of a gene, to simultaneously quantify all or a large proportion of the different mRNA species present in the cell at a certain point in time thereby characterizing the transcriptome of that cell. The term "response profile" indicating the composition of mRNA species before the stimulus minus the composition of mRNA species after the stimulus, corrected for general test influences.

In principle, any organism may be used in the methods of the present invention including plants, animals (including human) and preferably micro-organisms. Micro-organismen useful in methods of the present invention may include bacteria, archaea, fungi, yeast, protozoa and algae. More preferably bacteria, fungi and/or, yeast are used.

A "susceptible" organism in the context of the present description is an organism that is responsive to a chemical compound, i.e. that shows a detectable change in its chemical composition after exposure to said chemical compound. Thus, "susceptible" not only relates to growth inhibition by antibiotics, but is used herein in a more generalized context.

A "reference compound" is a chemical compound of which one or more characteristic used for classifying chemical compounds is known and that is used to establish reference response profiles. Known characteristics may include for instance the mode of action and/or target in case the compound is an antibiotic.

Suitable reference compounds include such antibiotics as erythromycin, tetracyclin and chloramphenicol as inhibitors of protein synthesis, colistin and polymyxin B sulphate as compounds interfering with membrane integrity, ciprofloxacin and rifampicin as compounds interfering with nucleic acid synthesis and ceftazidin and ampicillin as compounds interfering with cell wall synthesis.

The term "similarity" indicates the state or quality of being similar, i.e. of having the same or some of the same characteristics, such as mode of action, target, or biochemical response profile.

A known cluster of reference response profiles is a cluster of which the mode of action, target or cellular process affected is known. Such knowledge may be acquired from literature data or from empirical testing or from performing the methods of the present invention.

The term "affected" includes such meanings as acted upon, influenced, or altered, transformed, modified and/or changed. In the context of gene expression it may in particular indicate either an increase or decrease therein, whereas in the context of a cellular process it may in particular indicate an inhibition or stimulation thereof.

A "molecular target" is the cellular component of which the function is disturbed by the action of an antibiotic compound. Molecular targets known to the art include the peptidoglycan bridge in cell walls, the protein synthesis machinery in the form of the ribosome, and many proteins such as RNase P, dihydrofolate reductase and DNA gyrase. A mode of action of an antibiotic is for instance interference with protein synthesis, interference with membrane integrity or cell wall synthesis.

In general, the prior art approach of identifying targets of antibiotics is characterized by first identifying genes whose expression is affected and inferring from the thus obtained genetic information the possible targets or modes of action.

Conversely, the approach of the present invention is characterized by producing a large number of biochemical reference response profiles in combination with known modes of action, known targets and/or known metabolic routes affected, then analysing those profiles for common characteristics, i.e. clustering said profiles, and once a unique profile for a certain compound is found amongst the known clusters investigating said profile to yield the genetic information required for establishing e.g. the new metabolic route or new target. This approach is much more efficient and cost effective than the methods of the prior art.

A method of the present invention comprises the selection of potential antibiotic compounds by using classical microbiological techniques of testing growth inhibition of a target organism by a chemical or biological compound. Preferably said compound tests negative in a cytotoxicity test in mammalian cells and may thus potentially be used as an antibiotic. Growth inhibition of the organism indicates the susceptibility of the organism to the compound.

The selected potentially antibiotic compounds are than applied to generate biochemical response profiles as follows: The target organism is exposed to a high dose (higher than the Minimum Inhibitory Concentration or MIC) of the test compound, preferably in a mid log culture of said organism. After exposure for a duration of between 1 second and 24 hours, preferably between 1 and 20 minutes, the cellular metabolism in the cells of the organism is fixed or quenched. Quenching may suitably occur by using by immediately quenching the cellular metabolism with 4 volumes of 60% methanol at -40°C. Subsequently, the biochemical composition of the organism is determined. For instance, RNA molecules are isolated and labeled fluorescently in order to be detected by DNA micro-array hybridization. Said DNA micro-array representing for instance the genome of the organism. The hybridization results, i.e. in this case the transcription profiles, obtained with exposed cells are compared to the hybridization results obtained with normal, non-exposed cells that were cultured under identical conditions to provide transcription response profiles.

The transcription response profiles obtained after exposure of a susceptible organism to different compounds are preferably compared by principal component analysis which then provides for the clustering of transcription profiles according to e.g. similarity in biological mode of action or other similar parameters. The classification of compounds based on e.g. their mode of action is possible using the clusters thus obtained.

This classification of compounds is central to the approach of the present invention in that from this classification a multitude of possibilities arises. For instance, various classification schemes may be generated that are for instance based on the knowledge available on reference compounds such as knowledge on targets or modes of action or cellular processes affected. Alternatively, classification may be based purely on clustering of response profiles.

Based on one of the above methods of classifying, a compound may selected for further analysis. This analysis may comprise in a first instance the selection and identification of those genes whose expression is strongly affected in a certain cluster of transcription profiles. Based on these results it is determined which biological process in the cell is influenced by the compound. This information then forms the basis for selecting a limited group of potential molecular targets for the compound concerned.

In the search for new antimicrobial strategies, the classification of antibiotic compounds according to the present invention now facilitates identification of biochemical response profiles that are dissimilar to any known response profiles available in e.g. a database. From this, it is inferred that the response profile obtained is new and that either the target of the compound is new, or that the mode of action of the compound is new. Thus, by using the methods of the present invention one is able to search for new antibiotic compounds and/or to search for new targets

In practice the biochemical composition of an organism is the collection of measured data from biomolecules of organisms, preferably of micro-organisms, used in a method of the present invention. Said measured data are preferably obtained from qualitative measurements, but (semi)quantitative measurements may also be used.

A suitable collection of measured data is formed by the transcriptional expression profile of an organism, which may for instance be obtained by measuring the various messenger RNA molecules in the cell, i.e. the transcriptome. An alternative collection of measured data is formed by the protein expression profile of an organism obtainable by measuring the various protein molecules present in the cell, i.e. the proteome. Yet another alternative is the collection of metabolites, i.e. the metabolome.

The biochemical composition of an organism, which mirrors the biological condition or state of a cell, may thus suitably be measured by measuring the transcriptional state, whereby amounts of RNA are determined, the translational state, whereby amounts of protein are determined, the activity state, whereby enzymatic activities are determined, or the state of metabolic routes, whereby amounts of metabolites are determined, or by measuring combinations thereof. In fact, measurements of many different biomolecules may provide a biochemical composition of an organism that may be used in a methods of the present invention.

Such biomolecules may comprise polynucleotides, such as nucleic acids, (poly)peptides or proteins, polysaccharides, lipids, lipopolysaccharides and/or other cellular macro molecules. Also metabolic intermediates such as sugars, organic acids, alcohols, fatty acids, amino acids, nucleotides, and the like may be measured. In preferred embodiments of aspects of the present invention nucleic acids, such as RNA, including messenger, transfer and ribosomaal RNA or combinations thereof are measured.

In more preferred embodiments of the methods of the present invention the biochemical composition of an organism is determined by determining the transcriptional state of the cell, i.e. as measured in the form of messenger RNA.

Measurements of biomolecules of an organism that may be used for creating a collection of measured data that together provide a biochemical composition are preferably performed in the form of a parallel biochemical analysis method. Such measurements may for instance be performed by separating the cellular biomolecules, labeling them collectively or individually and detecting them qualitatively or quantitatively.

In this context, with separating the cellular biomolecules is meant the physical separation of groups of identical biomolecules on the basis of their specific biochemical features, with the aim of detecting and, if necessary quantifying the thus separated biomolecules. Physically separating the various groups of identical biomolecules is, however, not always necessary. In this context, with labeling of biomolecules is meant the specific or a-specific labeling of biomolecules with specific markers, with the aim of detecting and, if necessary quantifying the thus labeled biomolecules. Such techniques are well known to the art.

In order to perform such measurements use may be made of one or more techniques such as:
- *in situ* measurement techniques such as nucleic acid probe technology and/or immunological measurement techniques; using these techniques, various cellular constituents may be measured separately or individually, without factual separation thereof by using specific or a-specific detection techniques, the suitability of which depends on the substance to be detected;
- electrophoretic and/or chromatografic measurement techniques; using these techniques cellular constituents may be separated on the basis of *i.a.* size, weight, charge, sensitivity to denaturation, after which detection may occur by using specific or a-specific detection techniques, the suitability of which depends on the substance to be detected;
- micro array and/or DNA biochip techniques; using these techniques biochemical constituents are separated on the basis of affinity for a surface-immobilised binding partner whereafter detection may occur by using specific or a-specific detection techniques, the suitability of which depends on the substance to be detected.

Suitable detection techniques that may be applied in concert with the above techniques include autoradiographic detection techniques, detection techniques based on fluorescence, luminescence or phosphorescence or chromogenic detection techniques. These detection techniques are known in the art of detection of biomolecules.

Preferably in a method of the present invention the transcriptional state of an organism is measured by using techniques that involve hybridization to arrays of nucleic acid probes or nucleic acid mimicking probes.

In order to determine the transcriptional state of an organism the RNA, including both total RNA and mRNA, may be isolated from one or more cells of said organism. Every RNA isolation method capable of isolating mRNA may be used for such isolation procedure (see for instance Ausubel *et al.,* 1987-1993, *Current Protocols in Molecular Biology,* John Wiley & Sons. Inc. New York). Large amounts of samples may be treated by using methods known to the skilled person, such as the single-step isolation-process of Chomczynski (1989, U.S. Pat. No. 4,843,155).

Transcripts (the RNA copies that are the initial step in decoding the genetic information) represent RNA of differentially expressed genes, and may be identified in various samples by using a variety of mathods known to the skilled person. For instance, differential screening (Tedder *et al.,* 1988. Proc. Natl. Acad. Sci. USA 85:208-212), subtractive hybridization (Hedrick *et al.,* 1984, Nature 308:149-153; Lee *et al.,* 1984, Proc. Natl. Acad. Sci. USA 88:2825), differential display (Liang & Pardee, 1992, Science 257:967-971; U.S. Pat. No. 5,262,311) expressed sequence tag (EST) (Adams *et al.,* 1991, Science 52:1656), serial analysis of gene expression (SAGE) (Kinzler *et al.,* U.S. Pat. No. 5,695,937) or cDNA AFLP (Vos *et al.,* 1995, Nucleic Acids Res., 23, 4407-14) techniques may be used.

The identification of such differentially expressed genes may then be used to design specific markers or labels that can be used to determine in an expeditious manner the presence of particular (levels of) mRNA species that indicate the type of stimulus that the organism receives.

In order to measure the various mRNA molecules in an organism the mRNA is preferably first converted into complementary DNA (cDNA), for which known techniques are at the disposal of the skilled person, such as for instance reverse transcription if the mRNA into cDNA by using the enzyme reverse transcriptase. Optionally, the conversion of RNA into cDNA may be combined with amplification of the cDNA by PCR (Mullis 1987, U.S. Pat. No. 4,683,195, 4,683,202, en 4,800,159) - optionally in a nested, multiplex or asymmetrical format - or by using the ligase chain reaction (Barany 1991, Proc. Natl. Acad. Sci. USA 88:189-193; EP Application No., 320,308), self sustained sequence replication (3SR) (Guatelli *et al.,* 1990, Proc. Natl. Acad. Sci. USA 87:1874-1878), strand displacement amplification (SDA)" (U.S. Pat. Nos. 5,270,184, and 5,455,166), transcriptional amplification system (Kwoh *et al.,* Proc. Natl. Acad. Sci. USA 86:1173-1177), Q-Beta Replicase (Lizardi *et al.,* 1988, Bio/Technology 6:1197), Rolling Circle Amplication (RCA) or any other method for amplifying nuclei acids. In an alternative method, the RNA may also be directly used or be amplified prior to use by using available RNA amplification methods such as Nucleic Acid Sequence-Based Amplification (NASBA) (L. Malek *et al.,* 1994, Meth. Molec. Biol. 28, Ch. 36, Isaac PG, ed., Humana Press, Inc., Totowa, N.J.) or Transcription Mediated Amplification (TMA).

Methods for obtaining genetic information by using nuclei acid arrays are well known in the art (see i.a. *Chee et al.*, 1996, Science 274(5287):610-614).

In a method according to the present invention, the application of a DNA microarray is preferred. Such arrays of oligonucleotides comprise for instance DNA sequences that are specific for certain mRNA species or for genetic markers.

A DNA microarray may be produced by methods known to the skilled person. The manufacture and use of DNA microarray for the detection of specific nucleic acid sequences is described abundantly (US 5,571,639; Sapolsky *et al.,* 1999, Genet. Anal.-Biomolecular Eng. 14, 187-192; Shena *et al*., 1995, Science 270, 467-470; Sheldon *et al.,* 1993, Clinical Chem. 39, 718-719; Fodor *et al.,* 1991, Science 251, 767-773).

The skilled person is capable of producing or acquiring arrays to his own design and the accompanying reading equipment from specilized suppliers (for instance Affymetrix Corp., Santa Clara, CA, USA for DNA arrays and Ciphergen Biosystems, Fremont, CA, USA for Protein Chip Arrays).

By comparing the biochemical compositions before and after exposure to a chemical compound, and correcting for a general test influence with a control treatment, a biochemical response profile of that organism relating to that specific chemical compound may be obtained. A multitude of such biochemical response profiles may be stored in a database, so that the result of a measurement (a new biochemical response profile) may be easily compared to the available data. As a result, the database itself will grow in size and detail, making for better interpretations of forthcoming experiments.

By clustering some or all of the biochemical response profiles in the database with a newly acquired biochemical response profile, or by defining by computational analysis the mathematical relationship that defines a certain type of clustering of some or all of the biochemical response profiles in the database and then defining a mathematical formula that defines the position of the individual profiles in said type of clustering and applying that mathematical formula to the newly acquired biochemical response profile, one is able to determine the clustered position of said newly acquired biochemical response profile, amongst the known profiles. If said clustered position is within a known cluster, i.e. a cluster that comprises reference compounds of which the mode of action, target, cellular process affected, or any other classifiable characteristic is known, then one can assign that same characteristic to that compound with which the newly acquired biochemical response profile was obtained.

In order to strengthen this predictive character of the database or, in general, this classification scheme, the presence of the characteristic may be determined empirically.

If the clustered position of a newly acquired biochemical response profile is outside a known cluster, then one cannot assign a characteristic to that compound, yet the compound has potentially unknown characteristics, both intrinsically, and in terms of its effect on cells of organisms. Thus, such compounds make for interesting objects of study and their effect on cells may be analysed in more detail.

In principle, any type of analysis may be performed to gain more insight in the target, mode of action or cellular process affected by such a compound.

The present invention now provides for one such a method wherein a mode of action of an antibiotic compound is analysed. The analysis starts with the determination of the clustered position in a classification scheme of the transcription response profile generated with said compound. In order to analyse a new mode of action said compound should generate a transcription response profile whose clustered position is outside any known cluster of reference response profiles. In order to analyse an intrinsically known, but not yet analysed mode of action said compound should generate a transcription response profile whose clustered position is within a known cluster of reference response profiles. Irrespective of whether said clustered position corresponds to a known cluster of reference response profiles or is outside any known cluster of reference response profiles, the transcription response profile indicates the changes that have occurred in the pool of messenger RNAs as a result of exposure of the test organism to the compound. Thus, the expression of some genes is affected as a result of the exposure and by determining the spots on the micro-array that are substantially increased or decreased in intensity, the affected genes may be identified. Not all genes the expression of which is affected will be of equal relevance to the analysis of the mode of action of the compound. In stead, those genes that characterize the clustered position, which will be the genes that mainly determine the distinction between the specific experimental condition and the control condition - such as can be identified by principle component analysis - are of major importance. If multiple genes involved in a specific process are affected, then by analysing the combination of genes affected, one can infer the cellular process affected by the antibiotic compound, a clustered position outside any known cluster indicating a new process affected. If the analysis indicates that the expression of a number of genes, involved in one cellular process, is changed under influence of the compound tested, this would point to said process as being affected by the compound. For instance genes involved in protein synthesis would indicate that the cellular process of protein synthesis is affected, indicating that the mode of action is interference with cellular protein synthesis. Another indicator will be genes that show a specific response towards one compound, but not to other treatments or compounds.

In yet a further aspect the present invention provides a method of identifying the molecular target of an antibiotic compound. The first step in such a method is the performance of a method of analysing a mode of action of an antibiotic compound as described above (clustered position within known clusiters) or performing a method of identifying a new mode of action of an antibiotic compound as described above (clustered position outside known clusters). Such a method indicates the cellular process affected. Certain key enzymes or other cellular components that characterize said affected process are noted to form a group of potential molecular targets affected by the antibiotic compound. Subsequently, one can zoom in on that group, for instance by using antibody labels in a method of enzyme quantification, to identify the molecular target of the antibiotic compound.

The present invention also provides a method of identifying a new metabolic route. Essentially this method comprises the performance of a method of classifying a chemical compound as described above and determining for said compound that the clustered position is outside any known reference response profiles. As such, it is established that the cellular response to the compound was not seen before with other compounds. Then, the cellular process affected by said chemical compound is determined, essentially by using a method of identifying the new mode of action of said compound as described above. If said cellular process is a process which was not previously known to be affected, said process will involve a new metabolic route.

### EXAMPLES

### Example 1. Determination of antibiotic treatment by measuring the RNA expression of Pseudomonas putida

In the present test design, the *Pseudomonas putida* S12 strain was exposed to high doses of different antibiotic compounds, each administered at a concentration which is well beyond the MIC-value (Minimal Inhibitory Concentration) known for such antibiotic compounds. The cells were fixed, after which RNA was isolated from the cells. The isolated RNA was hybridized on an array coated with genomic DNA fragments from *Pseudomonas putida* S12. The data analysis involved normalization and analysis according to principal component analysis (PCA). The test was carried out in duplicate and repeated once.

### Bacterial strain and growth conditions

*Pseudomonas putida* S12 pure culture was obtained by plating on Trypton Soy Agar (TSA, Oxoid) followed by incubation at 30°C for 20 hours. After this, the strain was grown in TSB for 1 day at 30°C in order to obtain a pre-accumulation.

### Antibiotic treatment

*P. putida* S12 cultures that are in a specific physiological state as a result of antibiotic treatment were obtained by diluting an overnight accumulation of *P. putida* S12 (35°C) 20 times in 50 ml fresh TSB medium in Erlenmeyer flasks. In this manner, 4 simultaneous cultures of 50 ml were produced that were allowed approximately 7 hours to grow to an OD₆₆₀ of approximately 0.5 at 35°C. This was verified by measuring OD₆₆₀ of one culture every 30 minutes. At the moment the OD₆₆₀ was 0.5, the two other cultures were subjected to antibiotic treatment (compounds used in this experiment were erythromycin, tetracycline, colistin and polymyxin B sulphate). The other culture remained as control treatment. After 10 minutes, the cells from the three incubated cultures were quenched and harvested for RNA isolation.

Erythromycine and colistin were tested in several independent experiments.

### RNA isolation

Quenching of metabolic activity in the culture was achieved by spraying the 10 ml culture in a stirred solution of a cold 60% methanol solution in water that was kept at -40°C (essentially as described by W. de Koning & K. van Dam 1992. Anal. Biochem. 204:118-123). Then the cells were pelleted by 10-minute centrifugation at 4500 g at -20°C. The pellet was further treated at -45°C or stored at -80°C. RNA was essentially isolated as described by Chin-Yi & Wilkins (1994. Anal. Biochem. 223:7-12). A 2-ml screw-cap Eppendorf tube was filled with 0.4 grams of zirconium beads and 500 µl Trizol (Invitrogen, GibcoBRL) at 0°C. The cell pellet (-45°C) was resuspended in 450 µl borate buffer (Chin-Yi & Wilkins, 1994) at 37°C and then added to the Eppendorf tube with beads and Trizol, mixed by hand and put on ice. The cells were broken up by beating them for 60 seconds in the Beadbeater (Biospec Products). The suspension was put on ice, after which 100 µl chloroform was added, vortexed and then centrifuged at 12,000 g at room temperature. The water phase was extracted using 500 µl phenol/chloroform (1/1) and then using chloroform with interim centrifugation in order to obtain a pure water phase. To the water phase 0.4 volumes of isopropanol were added and 0.4 volumes of 0.8M NaCitrate, 1.2M NaCl, mixed and cooled on ice for 10 min. After that, RNA was pelleted by centrifugation at 20,000 g at 4°C. The pellet was washed with 70% ethanol of -20°C and after centrifugation (20,000 g at 4°C) dried under vacuum. The pellet was dissolved in 100µl water.

### Construction of microarray

To make genome-wide analysis of gene expression in *Pseudomonas putida* S12 possible, a genomic bank of this organism was made. For this purpose, 50 micrograms of genomic DNA were incorporated in TE buffer with 20% glycerol and sheared for 30 seconds at 1 bar in a nebulizer. The average fragment size was estimated at 2-3 kb by means of gel electrophoresis. After precipitation, the ends were blunted in the presence of T4 DNA polymerase, Klenow polymerase and dNTPs. After heat inactivation of the enzymes, the ends were phosphorylized using T4 polynucleotide kinase. After heat inactivation, the mixture was again separated on an agarose gel and fragments between 2 and 3 kb were cut from the gel and were purified using a Qiagen gel extraction kit. The fragments obtained in this manner were ligated in vector pSMART according to the "Clone Smart Blunt cloning kit" protocol (Lucigen Corp.). A part of this ligation mixture was transformed in *E*. *coli* ElectroMax DH10B cells (Invitrogen) and plated on ampicillin plates. Using a colony picker, these colonies were picked and transferred into 96-well microtiter plates. After overnight growing at 37°C, glycerol was added to a final concentration of 15% and the glycerol stocks were stored at -80°C.

The genomic inserts from this bank were multiplied using PCR amplification in 96-well PCR plates. For this purpose, the following components were put together: 5 microliters 10x SuperTaq buffer; 5 microliters 2 mM dNTP (Roche Diagnostics); 1 microliter primer L1; 1 microliter primer R1; 37 microliters MQ water; 1 microliter glycerol stock; 1.5 U SuperTaq polymerase. Primer L1 has the following base sequence: 5'-CAG TCC AGT TAC GCT GGA GTC -3'. Primer R1 has the following base sequence: 5'-CTT TCT GCT ATG GAG GTC AGG TAT G -3'. Both primers contain a free NH2 group at the 5'-end followed by a C6 linker. The following PCR program was used for amplification: 4 min at 94°C; 30x (30 sec at 94°C, 30 sec at 50°C, 3 min at 72°C); 10 min at 72°C; 4°C.

Following the reactions, the products were transferred to round-bottom 96-well plates and precipitated using isopropanol. The PCR products purified in this manner were transferred to 384-well plates, in which the DNA, after evaporating to dryness, was incorporated in 10 microliters 3xSSC. These spot plates were used to transfer a total of 5500 PCR products to microarray slides on which they were spotted in a regular and ordered pattern. After spotting, these microarrays were blocked, after which they were ready for further use in hybridization experiments.

### Labeling RNA

For the fluorescent labeling of total RNA, 12.5 micrograms of RNA were used. To this RNA, 0.5 microliters RNAsin, 1.25 micrograms of random primer (p(dN)6; Roche Diagnostics) and water were added to a final volume of 5 microliters. This mixture was incubated for 10 min at 70°C, then for 10 min at 20°C and then put on ice. Then the following components were added: 0.5 microliters RNAsin, 3 microliters 5X first strand buffer (SuperScriptII; Life Technologies), 1.5 microliters 0.1 M DTT, 3 microliters nucleotide mix (2.5 mM of dATP, dCTP, dGTP, 1 mM dTTP), 1 microliter Cy5-dUTP or Cy3-dUTP (Amersham Biosciences) and 1 microliter SuperScriptII enzyme (Life Technologies). After mixing, the reaction components were incubated for 2 hours at 42°C. Then the RNA was degraded by heating the samples for 2 min at 95°C, briefly spinning them down, adding 1.5 microliters fresh 2.5M NaOH and incubating this for 10 min at 56°C. By addition of 1.46 microliters 2.5M HAc, the reaction mixture is neutralized. This reaction mixture is purified by purification over an Autoseq G50 column (Amersham Biosciences) according to the accompanying directions. After this purification, 1/10 part of the labeled material is kept apart for spectrophotometric analysis.

### Prehybridization / Hybridization / Washing

In preparation for the hybridization, slides were laid in Petri dishes in 20 ml prehybridization solution (1% BSA, 5x SSC, 0.1% SDS, filtered through a 0.45-micrometer-filter) and rotated for 45 minutes at 42°C. Then the slide was dipped 5x in filter-sterilized milliQ water. Then the slide was washed again in pure milliQ water, after which the slide was dipped 5x in isopropanol. Then the slide was dried using a N₂ gun and was then ready for hybridization.

The samples labeled with Cy5-dUTP and with Cy3-dUTP that were compared in one hybridization were combined in one cup. To this, 4 microliters yeast tRNA (25 micrograms / microliter) were added, after which the mixture was evaporated to dryness. After drying, the pellet was incorporated in 30 microliters EasyHyb (Roche Diagnostics) and denaturated (95°C, 1.5 min) after spinning down briefly. The mixture was then pipetted onto the prehybridized slide, covered with a cover slip and introduced into a Corning Hybridization room and incubated overnight at 42°C.

After hybridization, the slide was removed from the hybridization room and directly introduced into 20 ml 1xSSC/0.2% SDS solution of 37°C. The slide was then transferred to a fresh 1xSSC/0.2% SDS solution of 37°C, the cover slip staying behind in the first washing solution. After vigorous agitation, the slide was transferred to a 0.5xSSC washing solution of 37°C and again well agitated. Then the slide was transferred to a 0.2x SSC washing buffer and mixed for 10 min at 20°C on a rotation platform at 300 rpm. After replacing the washing buffer, this step was repeated. Then the slide was dried by blowing away the residual liquid using a N₂ gun.

### Scanning and image analysis

After washing, the slide was stored in the dark or directly used for scanning. A quick scan with a resolution of 30 micrometers led to a selection of optimal scanning setting (ScanArray 4000, Perkin Elmer). The optimal scan of the Cy5 and Cy3 signal took place at a resolution of 10 micrometers and the resulting images were stored electronically. The images corresponding to a slide were opened in the software package ImaGene (version 4.2, BioDiscovery Inc.). After placing the grid, which represents the spot pattern of the DNA samples on the slides, and spot recognition, signals and background were calculated per spot. The data obtained were stored in electronic files and used for further data processing.

### Data pre-processing

After calculation of M and A values (Roberts *et al.,* 2000, Science, 287, 873-880; Dudoit *et al.,* 2000, Statistics, UC Berkeley, Technical reports #578), the raw data were normalized using Loess normalization (Cleveland *et al.,* 1991, Statistics and Computing, volume 1 (1) pp. 47-62), leaving the following spots out of account: 1) spots with overload (with signal intensity in one or both channels being greater than 64000); 2) spots with the background being greater than the signal; 3) reference spots; 4) spots that were assigned a different flag than 0 by the image analysis package.

In this experiment, only spots that were found to have a good signal for all experiments were taken into account. After Loess normalization, the individual array results were normalized to unity (sum of squares equal to 1).

### Data analysis

Then the dataset was analyzed using principal component analysis (PCA), with mean centering as the selected scaling method. Comparable results can be obtained using other scaling methods or using other multivariate methods, whether or not using the information in which clusters the different samples are present.

### Results

*P. putida* strain S12 was found to grow well or not in TSB depending on the amount and nature of the antibiotic used. For measuring cell response upon antibiotic treatment, an analysis of the gene expression pattern on the microarray after antibiotic treatment or control treatment was made. For this purpose, 10 minutes after antibiotic treatment, the cells were quenched, harvested and further processed for microarray analysis.

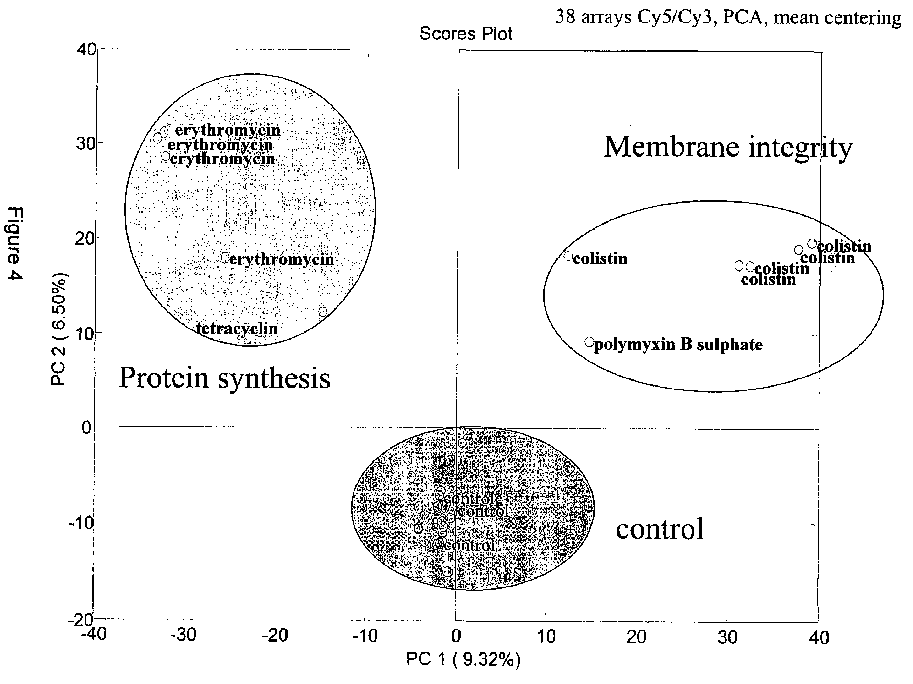

Figure 4 shows a score plot in which the data from the set of experiments are depicted. The Figure shows a clear difference between the antibiotic treatments in the various experiments. The two axes represent the principal components that together describe a total of 72% of the total variance in the dataset.

## Claims

1. Method of producing a classification scheme for chemical compounds comprising clustering according to similarity the biochemical response profiles of an organism as determined by exposing said organism to a multitude of individual chemical compounds.

2. Method according to claim 1, wherein said clustering according to similarity comprises principal component analysis.

3. Method according to claim 1 or 2, wherein said chemical compounds are not cytotoxic to mammalian cells.

4. Method according to any one of the preceding claims, wherein said organism is susceptible to said individual chemical compounds.

5. Method according to any one of the preceding claims, wherein said chemical compounds are antibiotic compounds.

6. Method according to any one of the preceding claims, wherein said multitude of individual chemical compounds comprises reference compounds with known mode of action and wherein said profiles are reference response profiles clustered according similarity in mode of action.

7. Classification scheme for chemical compounds obtainable by a method according to any one of claims 1 to 6.

8. Method of classifying a chemical compound, comprising the steps of:
a) providing a classification scheme for chemical compounds by performing a method of any one of claims 1-5;
b) exposing an organism to said chemical compound;
c) determining the biochemical response profile of said organism, and
d) determining in said scheme the clustered position of said biochemical response profile.

9. Method of classifying an antibiotic compound according to mode of action comprising the steps of:
a) providing a classification scheme for chemical compounds by performing a method of any one of claims 1-5, wherein said chemical compound is an antibiotic compound, wherein said multitude of individual antibiotic compounds comprises reference compounds with known mode of action and wherein said profiles are reference response profiles clustered according similarity in mode of action;
b) exposing an organism to said antibiotic compound;
c) determining the biochemical response profile of said organism;
d) determining in said scheme the clustered position of said biochemical response profile, and
e) assigning to said antibiotic compound the mode of action of the corresponding cluster of reference response profiles or assigning to said antibiotic compound a new mode of action in case said position is outside any known cluster of reference response profiles.

10. Method of analysing a mode of action of an antibiotic compound comprising the steps of:
a) performing a method of classifying an antibiotic compound according to claim 9, wherein the biochemical response profile is a transcription response profile, thereby determining for said antibiotic compound the clustered position of the transcription response profile;
b) identifying from said transcription response profile the genes of said organism of which the expression is substantially affected by exposure to said antibiotic compound and which characterize said clustered position, and
c) determining from the identity of said genes the cellular process affected by said antibiotic compound.

11. Method of identifying a new mode of action of an antibiotic compound comprising the steps of:
a) performing a method of classifying an antibiotic compound according to claim 9, wherein the biochemical response profile is a transcription response profile;
b) selecting an antibiotic compound of which the corresponding transcription response profile has a clustered position outside any known cluster of reference response profiles;
c) identifying from the transcription response profile of said selected compound the genes of said organism of which the expression is substantially affected by exposure to said selected antibiotic compound and which characterize said clustered position, and
d) determining from the identity of said genes the cellular process affected by said selected antibiotic compound thereby identifying the new mode of action.

12. Method of identifying the molecular target of an antibiotic compound comprising the steps of:
a) performing a method according to claim 10 or 11;
b) selecting a group of potential molecular targets **characterizing** said cellular process affected by said antibiotic compound, and
c) identifying the molecular target of said antibiotic compound in said group.

13. Method of identifying a new metabolic route comprising the steps of:
a) performing the method according to claim 8, wherein the biochemical response profile is a transcription response profile, wherein said multitude of individual chemical compounds comprises reference compounds with known mode of action and wherein said profiles are reference response profiles clustered according similarity in mode of action;
b) selecting a chemical compound of which the corresponding transcription response profile has a clustered position outside any known cluster of reference response profiles;
c) identifying from said transcription response profile the genes of said organism of which the expression is substantially affected by exposure to said chemical compound and which characterize said clustered position, and
d) determining from the identity of said genes the cellular process affected by said chemical compound thereby identifying a new metabolic route.


SEQUENCE LISTING

<110> Nederlandse Organisatie voor Toegepast-Natuurwetenschappelijk Onderzoek
      TNO

<120> Method of classifying chemical agents and identifying
      cellular targets thereof

<130> P65800EP00

<140> 03078246.0
<141> 2003-10-13

<160> 2

<170> PatentIn Ver. 2.1

<210> 1
<211> 21
<212> DNA
<213> Artificial Sequence

<220>
<223> Description of Artificial Sequence: primer L1

<220>
<221> misc_feature
<222> (1)..(21)

<400> 1
cagtccagtt acgctggagt c                                           21


<210> 2
<211> 25
<212> DNA
<213> Artificial Sequence

<220>
<223> Description of Artificial Sequence: primer R1

<220>
<221> misc_feature
<222> (1)..(25)

<400> 2
ctttctgcta tggaggtcag gtatg                                       25